# EUROPEAN PATENT APPLICATION

(11) **EP 3 834 628 A1**
(43) Date of publication of application: **16.06.2021**
(21) Application number: 19847391.0
(22) Date of filing: 05.08.2019
(51) Int. Cl.: A23L 33/10, A61K 31/121, A61K 31/353, A61P 1/00, A61P 1/14

(54) **COMPOSITION FOR IMPROVING INTESTINAL ENVIRONMENT AND METHOD FOR IMPROVING INTESTINAL FLORA**

(30) Priority: 10.08.2018 JP 2018151542
(71) Applicant: Suntory Holdings Limited, Osaka 530-8203 (JP)
(72) Inventor: FUKIZAWA, Shinya, Soraku-gun, Kyoto 619-0284 (JP); YAMASHITA, Mai, Soraku-gun, Kyoto 619-0284 (JP); WAKABAYASHI, Kenichi, Soraku-gun, Kyoto 619-0284 (JP)
(74) Representative: Vossius & Partner Patentanwälte Rechtsanwälte mbB
(86) International application number: PCT/JP2019/030724
(87) International publication number: WO 2020/031957

(57) **Abstract**

The present invention aims to provide a novel composition for improving gut flora and a method of improving gut flora, capable of increasing the abundance ratio of probiotics in the gut flora. The present invention relates to, for example, a composition for improving gut flora containing isoxanthohumol and/or xanthohumol as an active ingredient.

## Description

### TECHNICAL FIELD

The present invention relates to a composition for improving gut flora. The present invention also relates to a method of improving gut flora. The present invention further relates to use of isoxanthohumol and/or xanthohumol for improving gut flora.

### BACKGROUND ART

It has been reported that the gut flora not only functions as a barrier to prevent intrusion of exogenous pathogens but also affects energy regulation, nutrient uptake, immune function, and the like of the host, and that the structure of the gut flora changes in relation to obesity, diabetes, aging, and the like. For example, constipation, diarrhea, inflammatory bowel disease, cancer, allergy, asthma, and the like have been reported as conditions or diseases associated with the gut flora.

Diet is a factor that affects the gut flora. Methods of regulating the gut flora in expectation of some health benefits include intake of probiotic products for consumption of microorganisms (probiotics) that beneficially affect the host (in particular, humans) and contribute to the health, and intake of prebiotic products for growth of probiotics. With regard to growth of probiotics, various reports have been made on bacteria such as bifidobacteria, lactic acid bacteria, and later-described *Akkermansia* bacteria. According to one report, controlling the abundance ratio of Firmicutes to Bacteroidetes is important because the abundance ratio of Firmicutes bacteria is high and the abundance ratio of opportunistic Bacteroidetes bacteria is low in the gut flora of obese people (Non-Patent Literature 1).

*Akkermansia* bacteria such as *Akkermansia muciniphila* are intestinal bacteria living in the human intestinal mucus layer, and are known to be abundant in non-obese humans but scarce in obese people. According to one report, *Akkermansia* bacteria are also scarce in obese mouse model and type 2 diabetic mouse model, as compared to a regular mouse (Non-Patent Literature 2).

According to recent reports, the followings relate to the onset of chronic inflammation or systemic disease: stress- or obesity-induced disruption of biological defense function, i.e., intestinal barrier function, of intestinal epithelial cells; and entry of pathogenic bacteria or lipopolysaccharides (LPS) that are endotoxins, associated with the disruption. Intake of *Akkermansia* bacteria has been reported to enhance the intestinal barrier function and decrease lipopolysaccharide (LPS) entry into the serum (Non-Patent Literature 2). Thus, growth of *Akkermansia* bacteria in the intestine is expected to enhance the intestinal barrier function. Enhancement of the intestinal barrier function is expected to suppress the onset of chronic inflammation. Intestinal growth of *Akkermansia* bacteria that are probiotics is regarded as being beneficial to maintaining and promoting the host's health, and is considered to be a condition with improved gut flora or intestinal environment.

Non-Patent Literature 3 shows that intake of antidiabetic drug "metformin" has an action to promote the growth of *Akkermansia* bacteria. However, metformin, which is a pharmaceutical product, has a risk of side effects such as lactic acidosis. Thus, there has been a demand for a substance that can be suitably used in daily eating habits in order to improve the gut flora and the intestinal environment.

Patent Literature 1 discloses an agent that promotes *Akkermansia* bacteria growth, the agent containing a plant-derived pentamer or higher molecular weight proanthocyanidin as an active ingredient. Patent Literature 1 also suggests that while both a pentamer or higher molecular weight proanthocyanidin (PP) and a low molecular weight (monomer to tetramer) procyanidin fraction (OP) with a lower degree of polymerization than that of PP were both found to have an anti-obesity action, the action to promote the growth of *Akkermansia* bacteria was found only in the pentamer or higher molecular weight proanthocyanidin (PP), and that a component showing an anti-obesity action does not necessarily show a gut flora improving action. Thus, it is difficult to determine whether a substance has a gut flora improving action based on its anti-obesity action.

Non-Patent Literature 4 reports that spent hops (hop extract lees) containing polyphenols have an intestinal anti-inflammatory action. However, Non-Patent Literature 4 is unclear about the influence of spent hops on the gut flora, and nowhere specifies an active ingredient.

### CITATION LIST

### - Patent Literature

Patent Literature 1: JP 2018-8911 A

### - Non-Patent Literature

Non-Patent Literature 1: Nature. 2006 Dec 21; 444 (7122) :1027-31.
Non-Patent Literature 2: Proc Natl Acad Sci USA. 2013 May 28; 110(22):9066-71.
Non-Patent Literature 3: Appl Environ Microbiol. 2014 Oct; 80 (19)
Non-Patent Literature 4: BMC Vet Res. 2014 Sep 4; 10:196.

### SUMMARY OF INVENTION

### - Technical Problem

The present invention aims to provide a novel composition for improving gut flora, capable of increasing the abundance ratio of probiotics in the gut flora. The present invention also aims to provide, for example, a method of improving gut flora.

### - Solution to Problem

As a result of extensive studies to solve the above problems, the present inventors found that isoxanthohumol and xanthohumol, which are types of polyphenols derived from hops, have an action to promote the growth of *Akkermansia* bacteria and show a gut flora improving action. Isoxanthohumol and xanthohumol also have another gut flora improving action to decrease the ratio of Firmicutes bacteria to Bacteroidetes bacteria. The above ratio tends to be higher in obese people. With regard to isoxanthohumol and xanthohumol, no reports have been made on their actions that were found in the present invention, such as an action to grow *Akkermansia* bacteria, an action to decrease the ratio of Firmicutes bacteria to Bacteroidetes bacteria, and other actions based on these actions, such as a gut flora improving action, an intestinal barrier function enhancing action, and an intestinal environment improving action.

Specifically, although not limited thereto, the present invention relates to a composition for improving gut flora, a method of improving gut flora, and the like described below.
(1) A composition for improving gut flora, containing: isoxanthohumol and/or xanthohumol as an active ingredient.
(2) The composition for improving gut flora according to (1) above, wherein the improving gut flora is to increase the abundance ratio of probiotics in the gut flora.
(3) The composition for improving gut flora according to (2) above, wherein the probiotics are *Akkermansia* bacteria.
(4) The composition for improving gut flora according to (1) above, wherein the improving gut flora is to decrease the abundance ratio of Firmicutes bacteria to Bacteroidetes bacteria (Firmicutes/Bacteroidetes).
(5) The composition for improving gut flora according to any one of (1) to (4) above, wherein the composition is used to enhance intestinal barrier function.
(6) The composition for improving gut flora according to any one of (1) to (5) above, wherein the composition is a food or beverage.
(7) The composition for improving gut flora according to any one of (1) to (6) above, wherein the composition is a beverage.
(8) The composition for improving gut flora according to (7) above, wherein the beverage is a tea-based beverage, a coffee beverage, an alcohol beverage, a non-alcoholic beer-taste beverage, a carbonated beverage, a functional beverage, a fruit and/or vegetable-based beverage, a lactic beverage, a soy milk beverage, or flavored water.
(9) The composition for improving gut flora according to any one of (1) to (8) above, wherein the composition is labeled with one or more of the following function claims: "adjusting the balance of intestinal bacteria", "maintaining good intestinal environment", "helping to maintain intestinal health", "enhancing intestinal barrier function", "increasing probiotics", "increasing useful bacteria", "regulating gut conditions", "alleviating gut discomfort", and "contributing to a better predisposition".
(10) A method of improving gut flora, including: feeding or administering isoxanthohumol and/or xanthohumol to a subject.
(11) Use of isoxanthohumol and/or xanthohumol for improving gut flora.

### - Advantageous Effects of Invention

The present invention can provide a novel composition for improving gut flora, capable of increasing the abundance ratio of probiotics in the gut flora. Intake of the composition for improving gut flora of the present invention can, for example, increase the abundance ratio of *Akkermansia* bacteria, which are probiotics, in the gut flora. Such an action to increase the abundance ratio of probiotics or the like can improve the gut flora, improve the intestinal environment, enhance the intestinal barrier function, or the like. The present invention can provide a method capable of increasing the abundance ratio of probiotics in the gut flora and improving the gut flora.

### BRIEF DESCRIPTION OF DRAWINGS

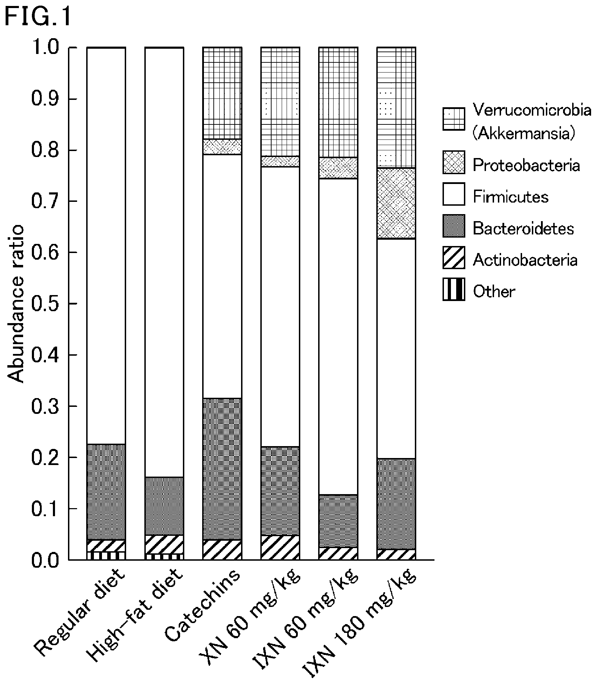

Fig. 1 is a graph showing the abundance ratio of bacteria in the gut flora of each group.

### DESCRIPTION OF EMBODIMENTS

The composition for improving gut flora of the present invention contains isoxanthohumol and/or xanthohumol as an active ingredient.

The composition for improving gut flora may contain one of isoxanthohumol or xanthohumol as an as active ingredient or may contain both of them as active ingredients.

In one embodiment of the present invention, preferably, the composition for improving gut flora contains isoxanthohumol as an active ingredient, or isoxanthohumol and xanthohumol as active ingredients.

Isoxanthohumol can be prepared, for example, through a process such as heating of a hop *(Humulus lupulus,* Cannabaceae) extract. Heating of a hop extract can produce isoxanthohumol in the extract. A hop extract is usually prepared through a process involving extraction of hop cones with a solvent and purification as needed. A hop extract can be obtained by a known preparation method. Hops can be extracted, for example, by a method that uses an ethanol solvent, which is used as a preparation method of a hop extract for beer brewing. A hop extract is commercially available, and a commercial hop extract can also be used. A hop extract is heated to produce isoxanthohumol preferably at 80°C to 140°C (more preferably 85°C to 100°C) for 15 minutes to 5 hours (more preferably 20 minutes to 3 hours). The hop extract is purified to prepare isoxanthohumol by a known method. Purification is performed by, for example, a method using HPLC or an absorption column or a precipitation method based on changes in solubility. Isoxanthohumol can also be produced by isomerizing xanthohumol by heating. Here, the heating temperature is preferably 80°C to 140°C (more preferably 85°C to 100°C) for 15 minutes to 5 hours (more preferably 20 minutes to 3 hours). Isoxanthohumol obtained by isomerization can be concentrated or purified by a known method (e.g., filtration, vacuum concentration, or lyophilization), as needed.

Xanthohumol is a component derived from hops, and is obtained by extracting from hops with a solvent. For example, dried hops are crushed into pellets, and immersed in an organic solvent such as an alcohol for extraction. Then, the obtained liquid extract is concentrated or dried, followed by isolation or purification by chromatography or the like, whereby xanthohumol can be obtained. The temperature for extraction, fractionation, and purification is preferably lower than 80°C, more preferably 5°C to 70°C, for example.

Commercially available isoxanthohumol and xanthohumol can also be used.

In the present invention, the composition of the present invention may contain a plant-derived material that abundantly contains isoxanthohumol and/or xanthohumol, as long as the effect of the present invention is achieved.

Isoxanthohumol and xanthohumol are compounds that are contained in natural products, foods, and/or beverages and that have been used as food ingredients. Thus, daily intake of isoxanthohumol and/or xanthohumol, for example, is considered to be unlikely to cause any problems in terms of safety. Thus, the present invention can provide a composition for improving gut flora which contains a highly safe component(s) as an active ingredient(s). Isoxanthohumol has been reported to be stable even at a high temperature of 100°C, for example. The Food Sanitation Act defines sterilization conditions as the standard of soft drinks and the like. For example, soft drinks having a pH of 4.0 or higher (excluding those having a pH of 4.6 or higher and a water activity higher than 0.94) need to be heated at 85°C for 30 minutes. In view of conversion of ingredients in such a sterilization process, isoxanthohumol is considered to be highly suitable for beverage applications.

In one embodiment, the present invention can provide various functional foods, functional beverages, and the like that show a gut flora improving action, that are thermally stable, and that contribute to maintaining and improving the health.

Isoxanthohumol and/or xanthohumol contained in the composition for improving gut flora can be quantified by, for example, high performance liquid chromatography (HPLC) or LC-MS/MS. Isoxanthohumol and xanthohumol are also advantageous in that they can be easily quantified and thus they can be easily used as active ingredients of foods with function claims and the like which require quantitative analysis and standardization of active ingredients.

In the present invention, the term "the gut flora" refers to an intestinal bacterial population (microflora). Bacteria in the gut flora include bacteria (probiotics) that beneficially affect the host (in particular, humans) and contribute to the health; pathogenic bacteria that produce harmful substances and adversely affect the body; bacteria that adversely affect the intestine when the resistance of the host is weakened; and bacteria that are neither probiotic nor pathogenic. Probiotics can also be referred to as "useful bacteria". The abundance ratio of various bacteria in the gut flora is sometimes referred to as "balance". The abundance ratio (balance) of bacteria in the gut flora changes due to factors such as eating habits, age, stress, and intake of medicines such as antibiotics. The gut flora balance affects the health of the host.

In the present invention, the expression "improving gut flora" and its equivalent expressions refer to changing the gut flora in a manner beneficial for the host (in particular, humans). The "improving gut flora" includes improving the gut flora balance. In the present invention, preferably, the "improving gut flora" is to increase the abundance ratio (abundance proportion) of probiotics in the gut flora. The expression "the abundance ratio of probiotics in the gut flora" means the occupancy of probiotics relative to the total bacterial count in the gut flora. Increasing the abundance ratio of probiotics in the gut flora usually changes indexes such as metabolites produced by the gut flora and intestinal pH in a manner beneficial for the host. As described above, the intestinal environment is improved by improved gut flora owing to an increase in the abundance ratio of probiotics, or by changes in indexes such as metabolites produced by the gut flora or intestinal pH in a manner beneficial for the host. The composition for improving gut flora of the present invention can be used as a composition for improving intestinal environment.

The abundance ratio of probiotics in the gut flora can be determined, for example, by 16S rRNA gene analysis of bacteria in feces.

Examples of probiotics include *Akkermansia* bacteria, bifidobacteria, and lactic acid bacteria. *Akkermansia* bacteria are preferred.

An increase in the abundance ratio of *Akkermansia* bacteria in the gut flora is one of indexes of improvement of the gut flora and improvement of the intestinal environment. As described above, increasing *Akkermansia* bacteria is expected to enhance the intestinal barrier function.

The term "intestinal barrier function" refers to a function to prevent the intrusion (permeation) of foreign materials (e.g., toxins such as endotoxin, inflammatory substances, and undigested products) into the body from the outside of intestinal epithelial cells (the inside of the intestine). A state where the intrusion of foreign materials into the body from the outside of intestinal epithelial cells is promoted as compared to a normal state is referred to as a state with increased permeability of foreign materials in intestinal epithelial cells. The phrase "to enhance the intestinal barrier function" means to suppress an increase in the permeability of foreign materials in intestinal epithelial cells and to decrease the permeability of foreign materials in intestinal epithelial cells. The intestinal barrier function enhancing effect is indicated by, for example, an increase in the electrical resistance value (transepithelial electric resistance: TEER) of intestinal epithelial cells or suppression of a decrease in the TEER. A person skilled in the art can select a specific method of evaluating the intestinal barrier function enhancing effect depending on the purpose.

In addition, as described above, the gut flora of obese people showed an increase in Firmicutes bacteria and a decrease in Bacteroidetes bacteria (Non-Patent Literature 1 described above). Thus, a decrease in the abundance ratio of Firmicutes bacteria to Bacteroidetes bacteria (Firmicutes/Bacteroidetes) is one embodiment of improving the gut flora and can be one of indexes of improvement of the intestinal environment. A decrease in the abundance ratio of Firmicutes bacteria to Bacteroidetes bacteria can be rephrased as an increase in the abundance ratio of Bacteroidetes bacteria to Firmicutes bacteria (Bacteroidetes/Firmicutes) .

As shown in Examples described later, the mice orally fed (orally administered) with isoxanthohumol or xanthohumol showed changes in the abundance ratio of bacteria in the gut flora and an increase in the abundance ratio of *Akkermansia* bacteria, as compared to mice not fed with isoxanthohumol or xanthohumol. Thus, isoxanthohumol and xanthohumol have an action to increase the abundance ratio of *Akkermansia* bacteria in the gut flora so as to improve the gut flora. Further, the mice orally fed with isoxanthohumol or xanthohumol showed a decrease in the abundance ratio of Firmicutes bacteria to Bacteroidetes bacteria (Firmicutes/Bacteroidetes). Thus, isoxanthohumol and xanthohumol have an action to decrease the abundance ratio of Firmicutes bacteria to Bacteroidetes bacteria (Firmicutes/Bacteroidetes).

The composition for improving gut flora of the present invention, which contains isoxanthohumol and/or xanthohumol as an active ingredient, can be used to increase probiotics, for example, the abundance ratio of *Akkermansia* bacteria, in the gut flora. Increasing the abundance ratio of *Akkermansia* bacteria is effective in enhancing the intestinal barrier function. Thus, the composition for improving gut flora of the present invention can be suitably used to enhance the intestinal barrier function. Enhancement of the intestinal barrier function is expected to produce the following effects: prevention or amelioration of chronic inflammation, prevention or amelioration of constipation, prevention or amelioration of diarrhea, alleviation of gut discomfort, contribution to a better predisposition, and the like.

In one embodiment, the composition for improving gut flora of the present invention can be used to decrease the abundance ratio of Firmicutes bacteria to Bacteroidetes bacteria (Firmicutes/Bacteroidetes).

The composition for improving gut flora of the present invention can be used, for example, to increase the abundance ratio of probiotics in the gut flora so as to improve the gut flora, to improve the intestinal environment, and/or to enhance the intestinal barrier function. The composition for improving gut flora of the present invention can also be used to decrease the abundance ratio of Firmicutes bacteria to Bacteroidetes bacteria in the gut flora so as to improve the gut flora and/or to improve the intestinal environment. In one embodiment, the composition for improving gut flora of the present invention can be used to prevent or ameliorate a condition or disease that can be effectively prevented or ameliorated by increasing the abundance ratio of probiotics, in particular, *Akkermansia* bacteria, in the gut flora. Examples of such a condition or disease include conditions or diseases that are expected to be prevented or ameliorated by enhancing the intestinal barrier function. Examples of the condition or disease that is expected to be prevented or ameliorated by enhancing the intestinal barrier function include chronic inflammation, constipation, diarrhea, irritable bowel syndrome, infection, and food allergy.

Herein, the expression "to prevent a condition or disease" refers to preventing the onset of a condition or disease, delaying the onset of a condition or disease, reducing the incidence of a condition or disease, reducing the onset risk of a condition or disease, and the like. The expression "to ameliorate a condition or disease" refers to helping a subject recover from a condition or disease, alleviating a symptom of a condition or disease, delaying or preventing the progress of a condition or disease, and the like.

The composition for improving gut flora of the present invention is applicable for either therapeutic use (medical use) or non-therapeutic use (non-medical use).

The composition for improving gut flora of the present invention can be provided, for example, as a food or beverage, a pharmaceutical or quasi-pharmaceutical product, or feed. The composition for improving gut flora of the present invention may be a food or beverage, a pharmaceutical or quasi-pharmaceutical product, feed, or the like by itself for improving gut flora; or may be a material, a preparation, or the like to be added to such a product or the like.

For example, the composition for improving gut flora of the present invention may be provided as an agent or the like, but it is not limited thereto. The agent can be provided directly as a composition, or can be provided as a composition containing the agent. The composition for improving gut flora of the present invention can also be referred to as a gut flora improver.

In order to sufficiently obtain the effect of the present invention, preferably, the composition for improving gut flora of the present invention is a composition for oral ingestion. The composition for oral ingestion may be a food or beverage, a pharmaceutical or quasi-pharmaceutical product for oral administration, or feed, preferably a food or beverage, or a pharmaceutical product for oral administration, still more preferably a food or beverage.

The composition for improving gut flora of the present invention can contain optional additives and optional components, in addition to isoxanthohumol and/or xanthohumol, as long as the effect of the present invention is not impaired. Such additives and components can be selected according to the composition form or the like. Those that can be used generally in foods, beverages, pharmaceutical products, quasi-pharmaceutical products, feed, and the like can be used.

When the composition for improving gut flora of the present invention is provided as a food or beverage, a pharmaceutical or quasi-pharmaceutical product, feed, or the like, any common method can be used for the production. For example, the composition for improving gut flora which contains isoxanthohumol can also be produced by using a hop extract and heating the hop extract.

For example, the composition for improving gut flora of the present invention is provided as a food or beverage, a component usable in a food or beverage (e.g., a food material or an optional food additive) can be added to isoxanthohumol and/or xanthohumol to provide various types of foods or beverages. Non-limiting examples of the food or beverage include general foods and beverages, health foods, health beverages, foods with function claims, foods for specified health uses, and foods and beverages for the sick. The health foods, the foods with function claims, and the foods for specified health uses can be used in various forms of preparations such as fine granules, tablets, granules, powders, capsules, chewable tablets, syrups, liquids, and liquid foods.

In an exemplary preferred embodiment, the composition for improving gut flora of the present invention may be a beverage. For example, since isoxanthohumol is thermally stable, the composition for improving gut flora which contains isoxanthohumol is suitable for a beverage.

The beverage may be a non-alcohol beverage or an alcohol beverage. Examples of the non-alcohol beverage include tea-based beverages, coffee beverages, non-alcoholic beer-taste beverages, carbonated beverages, functional beverages, fruit and/or vegetable-based beverages, lactic beverages, soy milk beverages, and flavored water.

When the composition for improving gut flora of the present invention is a beverage, preferably, it is a tea-based beverage, a coffee beverage, an alcohol beverage, a non-alcoholic beer-taste beverage, a carbonated beverage, a functional beverage, a fruit and/or vegetable-based beverage, a lactic beverage, a soy milk beverage, or flavored water.

When the composition for improving gut flora of the present invention is a tea-based beverage, preferably, it is a black tea beverage or a sugarless tea beverage. Examples of the sugarless tea beverages include green tea beverages, oolong tea beverages, barley tea beverages, brown rice tea beverages, adlay tea beverages, and sugarless black tea beverages.

When the composition for improving gut flora of the present invention is a coffee beverage, preferably, it is a packaged coffee beverage or liquid coffee.

Examples of the alcohol beverage include beer, beer-based beverages, and alcohol beverages other than the beer and beer-based beverages.

When the composition for improving gut flora of the present invention is a beer-based beverage, preferably, it is low-malt beer or a beer-like beverage.

When the composition for improving gut flora of the present invention is an alcohol beverage other than the beer and beer-based beverages, preferably, it is *shochu,* a *shochu* highball, a liqueur, cocktail, a spirit, or a whisky.

The term "non-alcoholic beer-taste beverage" as used herein refers to carbonated beverages with beer-like flavors, which are usually non-fermented non-alcohol beverages, substantially free of alcohols. Here, the non-alcoholic beer-taste beverage does not exclude beverages containing a trace amount (undetectable amount) of alcohol.

When the composition for improving gut flora of the present invention is a carbonated beverage, preferably, it is a cola-flavored beverage, a clear carbonated beverage, ginger ale, a fruit juice-based carbonated beverage, a milk-containing carbonated beverage, or a sugarless carbonated beverage.

When the composition for improving gut flora of the present invention is a functional beverage, preferably, it is a sports drink, an energy drink, a health-supporting beverage, or a jelly drink pouch.

When the composition for improving gut flora of the present invention is a fruit and/or vegetable-based beverage, preferably, it is a 100% fruit juice, a fruit-containing beverage, a soft drink with a low fruit juice content, a pulp-containing fruit juice, or a pulp-containing beverage.

When the composition for improving gut flora of the present invention is a lactic beverage, preferably, it is milk, a yogurt drink, a lactic acid bacteria beverage, or a milk-containing soft drink.

When the composition for improving gut flora of the present invention is a soy milk beverage, preferably, it is soy milk or a soybean beverage.

The form of the beverage is not particularly limited. Examples include packaged beverages. Packages for the packaged beverages are not particularly limited. Packages in any form and of any material may be used. For example, any of the following commonly used packages can be used: metal packages such as aluminum cans and steel cans; resin containers such as plastic bottles; paper containers such as drink cartons; glass containers such as glass bottles; and wooden containers such as barrels. The beverage is filled and sealed in any of these packages, whereby a packaged beverage can be obtained.

When the composition for improving gut flora of the present invention is provided as a pharmaceutical or quasi-pharmaceutical product, various dosage forms of pharmaceutical or quasi-pharmaceutical products can be provided by, for example, adding a pharmacologically acceptable carrier, an optional additive or the like to isoxanthohumol and/or xanthohumol. Such a carrier, an additive, or the like may be of any pharmacologically acceptable type that can be used in pharmaceutical or quasi-pharmaceutical products. Examples thereof include excipients, binders, disintegrants, lubricants, antioxidants, and colorants. One or more of these can be used. The form of administration (intake) of the pharmaceutical or quasi-pharmaceutical product may be an oral, enteral, or transmucosal administration or injection. Oral administration is preferred in order to sufficiently obtain the effect of the present invention. When the composition for improving gut flora is a pharmaceutical product, preferably, it is a pharmaceutical product for oral administration. Examples of dosage forms of preparations for oral administration include liquids, tablets, powders, fine granules, granules, sugar-coated tablets, capsules, suspensions, emulsions, and chewable tablets. The pharmaceutical product may be a pharmaceutical product for non-human animals.

When the composition for improving gut flora of the present invention is provided as feed, for example, isoxanthohumol and/or xanthohumol may be added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of isoxanthohumol and/or xanthohumol in the composition for improving gut flora of the present invention is not limited, and can be set according to the composition form or the like. The amount of isoxanthohumol and/or xanthohumol, for example, in terms of the total amount of isoxanthohumol and xanthohumol, in the composition for improving gut flora is preferably 0.0001 wt% or more, more preferably 0.001 wt% or more and is preferably 90 wt% or less. In one embodiment, the amount of isoxanthohumol and/or xanthohumol, in terms of the total amount of isoxanthohumol and xanthohumol, in the composition for improving gut flora is preferably 0.0001 to 90 wt%, more preferably 0.001 to 90 wt%. The total amount of isoxanthohumol and xanthohumol is the amount of isoxanthohumol or xanthohumol when the composition contains only one of them, and is the total amount of isoxanthohumol and xanthohumol when the composition contains both of them. In one embodiment, when the composition for improving gut flora of the present invention is provided as a food or beverage, a pharmaceutical or quasi-pharmaceutical product, feed, or the like, preferably, the total amount of isoxanthohumol and xanthohumol is in the above ranges.

The composition for improving gut flora of the present invention can be fed or administered by an appropriate method according to the form. Preferably, the composition for improving gut flora of the present invention is orally fed (orally administered) in order to sufficiently obtain the effect of the present invention.

The intake (dosage) of the composition for improving gut flora of the present invention is not limited, as long as it is the amount (effective amount) that provides the gut flora improving effect. The intake may be suitably set according to the dosage form, administration method, body weight of a subject, and the like. In one embodiment, when the composition for improving gut flora is orally fed or administered to a human (adult), the intake of the composition in terms of total intake of isoxanthohumol and xanthohumol is preferably 1 to 200 mg, more preferably 5 to 60 mg, per 60 kg per day. Intake of the above amount in one or more portions per day, for example, one to several times (e.g., two to three times) per day, is preferred. The total intake of isoxanthohumol and xanthohumol is the intake of isoxanthohumol or xanthohumol when the composition contains only one of them, and is the total intake of isoxanthohumol and xanthohumol when the composition contains both of them. In one embodiment, the composition for improving gut flora of the present invention may be a composition for oral administration to be used to feed or administer the above amount of isoxanthohumol and/or xanthohumol per 60 kg body weight per day to an adult.

A greater gut flora improving effect is obtained by continuous intake (administration) of isoxanthohumol and/or xanthohumol. Thus, in a preferred embodiment, the composition for improving gut flora of the present invention is fed continuously. In one embodiment of the present invention, the composition for improving gut flora is preferably fed continuously for two weeks or longer, more preferably for eight weeks or longer.

A subject to be fed (administered) with the composition for improving gut flora of the present invention is not limited. The subject is preferably a human or non-human mammal, more preferably a human.

The subject to be fed with the composition for improving gut flora of the present invention is preferably one who needs or wants to improve the gut flora or one who needs or wants to improve the intestinal environment. Examples of such a subject include people with irritable bowel syndrome, constipation, diarrhea, and/or food allergy. The composition for improving gut flora of the present invention can also be used on a healthy person, for example, for preventing a condition or disease that is expected to be prevented or ameliorated by improving the gut flora.

The composition for improving gut flora of the present invention contains a highly safe component that is contained in natural products and/or foods or beverages as an active ingredient, and exhibits a high level of gut flora improving effect when fed continuously. Thus, the composition for improving gut flora of the present invention is useful as a health food, a food with function claims, or the like used to increase the abundance ratio of probiotics in the gut flora, so as to improve the gut flora and/or the intestinal environment of the above described people with irritable bowel syndrome, constipation, diarrhea, or food allergy, or healthy people, for example, through daily oral intake.

The composition for improving gut flora of the present invention may be labeled with function claims based on the gut flora improving action. The composition for improving gut flora of the present invention may be labeled with one or more of the following function claims, for example: "adjusting the balance of intestinal bacteria", "maintaining good intestinal environment", "helping to maintain intestinal health", "enhancing intestinal barrier function", "increasing probiotics", "increasing useful bacteria", "regulating gut conditions", "alleviating gut discomfort", and "contributing to a better predisposition".

In one embodiment of the present invention, preferably, the composition for improving gut flora of the present invention is a food or beverage with one or more of the above functional claims. The labels may be labels indicating use for obtaining these functions.

The present invention also encompasses the following methods:
a method of improving gut flora, including feeding or administering isoxanthohumol and/or xanthohumol to a subject;
a method of improving intestinal environment, including feeding or administering isoxanthohumol and/or xanthohumol to a subject; and
a method of increasing the abundance ratio of probiotics in the gut flora, including feeding or administering isoxanthohumol and/or xanthohumol to a subject.

These methods may be therapeutic or non-therapeutic. The "non-therapeutic" is a concept that does not include medical activities, i.e., a concept that does not include surgery, therapy or diagnosis of humans.

Intake of isoxanthohumol and/or xanthohumol can increase the abundance ratio of probiotics in the gut flora. Intake of isoxanthohumol and/or xanthohumol can also decrease the abundance ratio of Firmicutes bacteria to Bacteroidetes bacteria (Firmicutes/Bacteroidetes) in the gut flora. These actions can improve the gut flora and the intestinal environment.

The present invention also encompasses the following uses:
use of isoxanthohumol and/or xanthohumol for improving gut flora;
use of isoxanthohumol and/or xanthohumol for improving intestinal environment; and
use of isoxanthohumol and/or xanthohumol for increasing the abundance ratio of probiotics in the gut flora.

Preferably, the uses are for a human or non-human mammal, more preferably for a human. The uses may be therapeutic or non-therapeutic.

In the methods and uses of the present invention, improving gut flora, probiotics, and preferred embodiments thereof are as described above for the composition for improving gut flora of the present invention.

The present invention also encompasses a method of increasing the abundance ratio of *Akkermansia* bacteria in gut flora, including feeding or administering isoxanthohumol and/or xanthohumol to a subject; a method of decreasing the abundance ratio of Firmicutes bacteria to Bacteroidetes bacteria (Firmicutes/Bacteroidetes) in gut flora, including feeding or administering isoxanthohumol and/or xanthohumol to a subject; use of isoxanthohumol and/or xanthohumol for increasing the abundance ratio of *Akkermansia* bacteria in gut flora; and use of isoxanthohumol and/or xanthohumol for decreasing the abundance ratio of Firmicutes bacteria to Bacteroidetes bacteria (Firmicutes/Bacteroidetes) in gut flora.

In the methods and the uses described above, preferably, isoxanthohumol and/or xanthohumol is continuously fed or administered to a subject in order to sufficiently obtain the effect of the present invention.

In the case of continuous feeding or administration of isoxanthohumol and/or xanthohumol to a subject, isoxanthohumol and/or xanthohumol is continuously fed or administered preferably for two weeks or longer, more preferably for eight weeks or longer, in order to more sufficiently obtain the effect of the present invention. In the methods and the uses described above, isoxanthohumol and/or xanthohumol is administered or fed to a subject in one or more portions per day, for example, one to several times (e.g., two to three times) per day. In one embodiment, preferably, the methods and the uses described above each include feeding or administering isoxanthohumol, or isoxanthohumol and xanthohumol.

In the methods and the uses described above, isoxanthohumol and/or xanthohumol is administered or fed to a subject in an amount (effective amount) that provides the gut flora improving effect. A preferred intake of isoxanthohumol and/or xanthohumol and a preferred subject to be administered with isoxanthohumol and/or xanthohumol are as described above for the composition for improving gut flora of the present invention. Isoxanthohumol and/or xanthohumol may be directly administered or fed, or may be administered or fed in the form of a composition containing isoxanthohumol and/or xanthohumol. For example, the composition for improving gut flora of the present invention described above may be administered or fed.

Isoxanthohumol and/or xanthohumol can also be used to produce a food or beverage, a pharmaceutical or quasi-pharmaceutical product, feed, or the like used to improve the gut flora. In one embodiment, the present invention also encompasses use of isoxanthohumol and/or xanthohumol for producing a composition for improving gut flora.

All academic literature and patent literature described herein are incorporated herein by reference.

### EXAMPLES

The present invention is described in further detail below with reference to examples, but the scope of the present invention is not limited thereto.

A series of animal experiments was performed based on a plan approved by the relevant chief through evaluation of the in-house animal experiment committee, in compliance with the animal welfare management laws and other related laws and regulations.

### <Preparation Example 1> Preparation of xanthohumol and isoxanthohumol

Isoxanthohumol and xanthohumol were isolated and purified from a hop extract (Asama Chemical. Co., Ltd.) by the following method. Specifically, using a hop extract as a raw material, isoxanthohumol and xanthohumol were purified by normal-phase column chromatography, reverse phase column chromatography, and preparative HPLC, and the purity was determined to be 95% or higher by HPLC analysis. For HPLC analysis, a Develosil C30-UG-5 column (Nomura Chemical Co., Ltd.) was used. The detector wavelength to measure the UV absorption was 280 nm (isoxanthohumol) or 350 nm (xanthohumol). The obtained isoxanthohumol and xanthohumol were used as standard samples (both having a purity of 95% or higher) in the following experiments.

### <Example 1>

Study on gut flora improving action by intake of xanthohumol and isoxanthohumol

The influence of xanthohumol and isoxanthohumol on the gut flora was examined by the following procedure using a high fat diet mouse model.

### (Group structure)

Table 1 shows groups (group name, basic feed, test substance, and dosage of the test substance) in the present test. The dosage is an intake (mg) of the test substance per kg body weight per day.

In the table, "Regular diet" is control diet "D12450J" (Research Diets), and "60 kcal% High-fat diet" is very high-fat diet "D12492" (Research Diets); "Catechins" is a preparation "Polyphenon 70A" (product name, Mitsui Norin Co., Ltd.). Catechins were used as a positive control. Xanthohumol and isoxanthohumol were the standard samples (purity of 95% or higher) obtained in Preparation Example 1.

**[Table 1]**

| Group No. | Group name | Basic feed | Test substance | Dosage (mg/kg/day) |
|---|---|---|---|---|
| 1 | Regular diet | Regular diet | N/A | 0 |
| 2 | High fat diet | 60kcal% High fat diet | N/A | 0 |
| 3 | Catechins | 60kcal% High fat diet | Catechins | 300 |
| 4 | Xanthohumol (XN) | 60 kcal% High fat diet | Xanthohumol | 60 |
| 5 | Isoxanthohumol (IXN) | 60 kcal% High fat diet | Isoxanthohumol | 60 |
| 6 | Isoxanthohumol (IXN) | 60 kcal% High fat diet | Isoxanthohumol | 180 |

### (Acclimatization and grouping)

Mice (C57BL/6J, male, 7 weeks of age, CLEA Japan, Inc.) were purchased, and they were quarantined and acclimatized for one week. Then, animals were selected based on changes in body weight from animals that showed no abnormalities in observation of general conditions. The selected animals were divided into groups (n = 8 in each group) in the group structure shown in Table 1 by the stratified continuous randomization method based on body weight at the end of the acclimatization period. They were fed with free access to specific basic feed and water.

### (Method of test substance intake)

The test substance was orally fed once a day for eight weeks. A dosing solution containing a mixture of the test substance and a solvent was used to administer the test substance. The solvent was a 0.5 wt% sodium carboxymethylcellulose (CMC) aqueous solution. The volume of the dosing solution to be administered to each mouse was 10 mL/kg. The amount of the test substance and the volume of the solution were calculated based on the latest body weight.

### (Collection of feces and gene analysis)

16S rRNA gene analysis was performed on feces (fresh feces) collected eight weeks after the start of intake of the test substance. 16S rRNA gene analysis was performed by the method described in Examples of JP 2018-8911 A. The abundance proportions (abundance ratio) of intestinal bacteria found in the feces were determined by the gene analysis as the abundance ratio of bacteria in the gut flora.

### (Results)

Fig. 1 is a graph showing the abundance proportion (group average) of bacteria in the gut flora of each group. The abundance proportion shown in Fig. 1 is the ratio (occupancy) of each species of bacteria to the total bacterial count in the feces. Verrucomicrobia in Fig. 1 is a higher rank to which *Akkermansia* bacteria belong. All the Verrucomicrobia bacteria in this analysis were *Akkermansia* bacteria. The dosage (mg/kg) of isoxanthohumol (IXN) and xanthohumol (XN) in Fig. 1 is the intake (mg) of the test substance per kg body weight per day. Individuals subjected to intake of xanthohumol or isoxanthohumol for eight weeks showed changes in the gut flora and an increase in the abundance proportion of *Akkermansia* bacteria, as compared to individuals not subjected to such intake (High fat diet group (Group No. 2) in Table 1). The same effects were observed in Catechins intake group. Individuals subjected to intake of xanthohumol or isoxanthohumol for the period described above showed a decrease in the abundance ratio of Firmicutes bacteria to Bacteroidetes bacteria, as compared to High fat diet group. The abundance ratio of Firmicutes bacteria to Bacteroidetes bacteria was as follows: 4.2 in Regular diet group; 7.4 in High fat diet group; 1.7 in Catechins group; 3.2 in Xanthohumol (60 mg/kg) group; 6.0 in Isoxanthohumol (60 mg/kg) group (Group No. 5); and 2.4 in Isoxanthohumol (180 mg/kg) group (Group No. 6).

### <Example 2>

Fig. 1 shows the results of intake of each test substance for eight weeks. Another test was performed using isoxanthohumol as the test substance, with partial modification in the group structure and dosage design. In this test, the cecal contents were collected two weeks after the start of intake of the test substance, and 16S rRNA gene analysis was performed in the same manner as described above. Two weeks after the start of intake of the test substance, Isoxanthohumol (60 mg/kg) group (group subjected to intake of 60 mg of isoxanthohumol per kg body weight per day) showed an increase in the abundance ratio of *Akkermansia* bacteria in the gut flora, as compared to individuals not subjected to such intake (High fat diet group) .

### INDUSTRIAL APPLICABILITY

The present invention is useful in the fields of foods, beverages, pharmaceutical products, and the like.

## Claims

1. A composition for improving gut flora, comprising:
isoxanthohumol and/or xanthohumol as an active ingredient.

2. The composition for improving gut flora according to claim 1,
wherein the improving the gut flora is to increase the abundance ratio of probiotics in the gut flora.

3. The composition for improving gut flora according to claim 2,
wherein the probiotics are *Akkermansia* bacteria.

4. The composition for improving gut flora according to claim 1,
wherein the improving the gut flora is to decrease the abundance ratio of Firmicutes bacteria to Bacteroidetes bacteria (Firmicutes/Bacteroidetes).

5. The composition for improving gut flora according to any one of claims 1 to 4,
wherein the composition is used to enhance intestinal barrier function.

6. The composition for improving gut flora according to any one of claims 1 to 5,
wherein the composition is a food or beverage.

7. The composition for improving gut flora according to any one of claims 1 to 6,
wherein the composition is a beverage.

8. The composition for improving gut flora according to claim 7,
wherein the beverage is a tea-based beverage, a coffee beverage, an alcohol beverage, a non-alcoholic beer-taste beverage, a carbonated beverage, a functional beverage, a fruit and/or vegetable-based beverage, a lactic beverage, a soy milk beverage, or flavored water.

9. The composition for improving gut flora according to any one of claims 1 to 8,
wherein the composition is labeled with one or more of the following function claims: "adjusting the balance of intestinal bacteria", "maintaining good intestinal environment", "helping to maintain intestinal health", "enhancing intestinal barrier function", "increasing probiotics", "increasing useful bacteria", "regulating gut conditions", "alleviating discomfort in gut", and "contributing to a better predisposition".

10. A method of improving gut flora, comprising:
feeding or administering isoxanthohumol and/or xanthohumol to a subject.

11. Use of isoxanthohumol and/or xanthohumol for improving gut flora.
